(19) Europäisches Patentamt
European Patent Office
Office européen des brevets

(11) **EP 1 285 256 B1**

(12) **FASCICULE DE BREVET EUROPEEN**

(45) Date de publication et mention
de la délivrance du brevet:
**09.07.2008 Bulletin 2008/28**

(51) Int Cl.:
**G01N 21/47** (2006.01) **A61B 5/00** (2006.01)

(21) Numéro de dépôt: **01936568.3**

(86) Numéro de dépôt international:
**PCT/FR2001/001520**

(22) Date de dépôt: **17.05.2001**

(87) Numéro de publication internationale:
**WO 2001/088507 (22.11.2001 Gazette 2001/47)**

(54) **PROCEDE D'ANALYSE D'UN ECHANTILLON DIFFUSANT PAR MESURE RESOLUE EN TEMPS**

VERFAHREN ZUR ANALYSE EINER STREUENDEN PROBE DURCH ZEITAUFGELÖSTE MESSUNG

METHOD OF ANALYSING A DIFFUSING SAMPLE BY TIME RESOLUTION MEASUREMENT

(84) Etats contractants désignés:
**AT BE CH CY DE DK ES FI FR GB GR IE IT LI LU MC NL PT SE TR**
Etats d'extension désignés:
**AL LT LV MK RO SI**

(30) Priorité: **18.05.2000 FR 0006392**

(43) Date de publication de la demande:
**26.02.2003 Bulletin 2003/09**

(73) Titulaire: **Université Paris XIII**
**93430 Villetaneuse (FR)**

(72) Inventeur: **TUALLE, Jean-Michel**
**F-92160 Antony (FR)**

(74) Mandataire: **Jacobson, Claude et al**
**Cabinet Lavoix**
**2, Place d'Estienne d'Orves**
**75441 Paris Cedex 09 (FR)**

(56) Documents cités:
- HEE M R ET AL: "FEMTOSECOND TRANSILLUMINATION OPTICAL COHERENCE TOMOGRAPHY" OPTICS LETTERS,US,OPTICAL SOCIETY OF AMERICA, WASHINGTON, vol. 18, no. 12, 15 juin 1993 (1993-06-15), pages 950-952, XP000345900 ISSN: 0146-9592
- WANG L ET AL: "TIME-RESOLVED FOURIER SPECTRUM AND IMAGING IN HIGHLY SCATTERING MEDIA" APPLIED OPTICS,US, OPTICAL SOCIETY OF AMERICA,WASHINGTON, vol. 32, no. 26, 10 septembre 1993 (1993-09-10), pages 5043-5048, XP000393405 ISSN: 0003-6935
- ZHANG T ET AL: "MULTIPASS MICHELSON INTERFEROMETER WITH THE USE OF A WAVELENGTH-MODULATED LASER DIODE" APPLIED OPTICS,US,OPTICAL SOCIETY OF AMERICA,WASHINGTON, vol. 35, no. 28, 1 octobre 1996 (1996-10-01), pages 5650-5656, XP000629793 ISSN: 0003-6935
- CHANCE B ET AL: "PHASE MEASUREMENT OF LIGHT ABSORPTION AND SCATTER IN HUMAN TISSUE" REVIEW OF SCIENTIFIC INSTRUMENTS,US,AMERICAN INSTITUTE OF PHYSICS. NEW YORK, vol. 69, no. 10, octobre 1998 (1998-10), pages 3457-3481, XP000830323 ISSN: 0034-6748
- HUANG D. ET AL: 'OPTICAL COHERENCE TOMOGRAPHY' SCIENCE, AMERICAN ASSOCIATION FOR THE ADVANCEMENT OF SCIENCE vol. 254, NEW YORK, US, pages 1178 - 1181, XP000604667

**Description**

La présente invention concerne un procédé d'analyse d'un échantillon diffusant par mesure résolue en temps de la lumière diffuse dans cet échantillon.

Dans le domaine médical, il est connu d'utiliser la lumière, notamment avec des longueurs d'ondes situées dans le domaine infrarouge, afin d'étudier assez profondément la nature des tissus biologiques.

Cependant, les tissus biologiques sont très diffusants aux longueurs d'ondes optiques, ce qui a tendance à brouiller toutes les informations concernant leur structure interne. Or, cette structure influe sur les données et il peut être nécessaire d'estimer cette influence. Pour cela, il est intéressant d'étudier la dynamique de propagation de la lumière dans les tissus biologiques. A cet effet, il est connu d'envoyer une impulsion lumineuse très courte sur le milieu à étudier et d'effectuer des mesures résolues en temps de la lumière diffuse. La résolution temporelle fournit des indications précieuses sur la structure du milieu.

Les mesures peuvent être effectuées en réflectance ou en transmittance.

Les mesures en réflectance sont utilisées notamment afin de déterminer le taux d'oxygénation de l'hémoglobine du sang.

Les mesures en transmittance sont utilisées notamment pour la détection des tumeurs cancéreuses du sein. Les images obtenues par la mesure en transmittance résolue en temps de la lumière diffuse produisent des images très contrastées permettant d'y déceler nettement une tumeur. Toutefois, la résolution spatiale des images est relativement médiocre.

Cette limitation provient de la technologie utilisée pour la mise en oeuvre de la mesure. En effet, la résolution temporelle nécessite d'être effectuée sur un temps très bref de l'ordre de quelques dizaines de picosecondes. Aussi, une telle résolution nécessite des technologies très sophistiquées et coûteuses.

Dans les dispositifs d'observation connus, la source produisant l'impulsion lumineuse est adaptée pour fournir une impulsion très brève.

Par exemple, dans le dispositif décrit dans le document US-5,692,511, la source lumineuse utilisée est un laser titane-saphir. Celui-ci est très coûteux. De plus, la détection mise en oeuvre est délicate. La solution utilisée dans le dispositif décrit dans ce document met en oeuvre une photodiode à avalanche reliée à une porte temporelle électronique afin de déterminer le nombre de photons transmis pendant une période de temps déterminée. La photodiode à avalanche a un temps de réponse relativement long qui détériore le contraste potentiel de la porte temporelle.

Plus généralement, dans les dispositifs d'analyse par mesure résolue en temps de la lumière diffuse, il est couramment mis en oeuvre une source permettant de produire des impulsions brèves telles que des lasers impulsionnels qui sont coûteux.

Alternativement, il est également connu d'utiliser une diode superlumiscente encombinaison avec un interféromètre de Michelson (voir D, Huang et Al., science, vol. 254, p.1178-1181, 1991).

De plus, les moyens mis en oeuvre pour la détection de la lumière posent des problèmes propres à chacun d'eux qui ne permettent pas une utilisation satisfaisante du dispositif.

Parmi ces moyens de détection du signal, on peut distinguer :

- les détecteurs rapides avec traitement électronique du signal provenant du détecteur dont le temps de réponse, relativement long, détériore considérablement le contraste de la porte temporelle ;
- les convertisseurs temps-amplitude en régime de comptage de photons qui nécessitent de diminuer le signal pour se placer dans un régime adapté, ce qui n'est pas compatible avec les temps d'acquisition courts ;
- les portes optiques à effet non linéaire qui nécessitent des lasers puissants à cadences faibles de l'ordre de 10 Hz, ce qui limite considérablement la sensibilité ;
- les caméras à balayage de fente qui possèdent une très bonne résolution temporelle (environ 2 ps) mais dont la dynamique n'est pas très importante : et
- les caméras intensifiées ultra-rapides dont la résolution temporelle n'est pas très bonne (de l'ordre de 80 ps) pour un faible taux de répétition (de l'ordre de 1 kHz).

Ainsi, les technologies actuellement disponibles ne permettent pas d'obtenir une bonne résolution spatiale, notamment en tomographie diffuse.

L'invention a pour but de proposer un dispositif d'analyse d'un échantillon diffusant par mesure résolue en temps de la lumière diffuse dont le coût de mise en oeuvre est modique.

A cet effet, l'invention a pour objet un procédé d'observation d'un échantillon diffusant du type précité, caractérisé en ce qu'il comporte les étapes de :

a) illuminer l'échantillon avec un faisceau incident de lumière cohérente temporellement et modulée en longueur d'onde ;

b) produire une succession de signaux d'interférence, enregistrés sur un intervalle de temps, par superposition de la lumière diffuse obtenue en sortie de l'échantillon et de lumière prélevée sur le faisceau incident illuminant l'échantillon ;

c) combiner chaque signal d'interférence avec un signal de référence générant une porte temporelle centrée sur un retard prédéterminé pour produire un signal caractéristique du retard prédéterminé ;

d) extraire la composante continue de chaque signal caractéristique du retard prédéterminé ;

e) appliquer une fonction non linéaire sur chacune des composantes continues des signaux caractéristiques du retard prédéterminé ; et

f) effectuer une combinaison linéaire de chacune des images, après application de ladite fonction non linéaire, des composantes continues des signaux caractéristiques du retard prédéterminé.

Suivant des modes particuliers de mise en oeuvre, le procédé comporte l'une ou plusieurs des caractéristiques suivantes :

- ladite fonction non linéaire est une fonction choisie dans le groupe consistant en l'élévation au carré, et la fonction valeur absolue ;

- ladite fonction non linéaire est une fonction à au moins deux variables prises parmi les composantes continues des signaux caractéristiques du retard prédéterminé obtenus à partir de la succession des signaux d'interférence enregistrés sur l'intervalle de temps ;

- pour la combinaison de chaque signal d'interférence avec ledit signal de référence, le faisceau incident est modulé, de façon supplémentaire, en amplitude au moyen d'une modulation en amplitude introduisant le retard prédéterminé ;

- pour une modulation en amplitude valant la somme dudit signal de référence et d'un signal de référence à retard nul, ladite combinaison linéaire comporte un calcul de soustraction de chacune des images, après application de ladite fonction non linéaire, des composantes continues des signaux caractéristiques d'un retard nul ;

- ladite combinaison linéaire comporte le calcul de la moyenne de chacune des images après application de ladite fonction non linéaire des composantes continues des signaux caractéristiques du retard prédéterminé ;

- pendant la production de la succession de signaux d'interférence, un déplacement relatif est imposé entre le faisceau incident et l'échantillon diffusant, la fréquence du déplacement imposé étant inférieure à la fréquence de la modulation en longueur d'onde de la lumière du faisceau incident ; et

- ledit signal de référence comporte une fonction sinusoïdale du produit du retard prédéterminé sur lequel est centrée la porte temporelle et de la fonction de modulation en longueur d'onde du faisceau incident.

L'invention a en outre pour objet une installation d'analyse d'un échantillon diffusant par mesure résolue en temps de la lumière diffuse dans cet échantillon, caractérisée en ce qu'elle comporte :

a) des moyens pour illuminer l'échantillon avec un faisceau incident de lumière cohérente temporellement et modulée en longueur d'onde ;

b) un interféromètre pour produire une succession de signaux d'interférence, enregistrés sur un intervalle de temps, par superposition de la lumière diffuse obtenue en sortie de l'échantillon et de lumière prélevée sur le faisceau incident illuminant l'échantillon ;

c) des moyens pour combiner chaque signal d'interférence avec un signal de référence générant une porte temporelle centrée sur un retard prédéterminé pour produire un signal caractéristique du retard prédéterminé ;

d) des moyens pour extraire la composante continue de chaque signal caractéristique du retard prédéterminé ;

e) des moyens pour appliquer une fonction non linéaire sur chacune des composantes continues des signaux caractéristiques du retard prédéterminé ; et

f) des moyens pour effectuer une combinaison linéaire de chacune des images, après application de ladite fonction non linéaire, des composantes continues des signaux caractéristiques du retard prédéterminé.

Elle a également trait à un équipement de mesure de la teneur en un constituant déterminé dans une partie d'un organisme vivant comportant une installation d'analyse d'un échantillon diffusant telle que décrite ci-dessus, et des moyens de calcul de ladite teneur en un constituant déterminé en fonction du résultat de l'analyse fourni par ladite installation.

Selon un mode particulier de réalisation, ladite installation d'analyse est adaptée pour produire des signaux d'interférence à partir de la lumière rétrodiffusée par un organe d'un être humain ou d'un animal, et lesdits moyens de calcul sont adaptés pour calculer le taux d'oxygénation de cet organe.

Enfin, l'invention a pour objet un équipement d'imagerie comportant :

- une installation d'analyse telle que décrite ci-dessus, laquelle installation est adaptée pour produire et traiter plusieurs successions de signaux d'interférence obtenus à partir de la lumière diffuse en plusieurs régions adjacentes de l'échantillon et ainsi fournir un résultat d'analyse pour chacune des régions adjacentes de l'échantillon ; et

- des moyens pour produire une image de l'échantillon à partir du résultat d'analyse fourni pour chacune des régions adjacentes de l'échantillon.

Selon un mode particulier de réalisation, ladite installation d'analyse est adaptée pour produire des signaux d'interférence à partir de la lumière diffuse transmise au travers d'un organe d'un être humain ou d'un animal.

L'invention sera mieux comprise à la lecture de la description qui va suivre, donnée uniquement à titre d'exemple, et faite en se référant aux dessins sur lesquels :

- la figure 1 est une vue schématique d'une installation d'analyse selon l'invention ;
- la figure 2 est un organigramme illustrant la structure de l'unité de traitement d'informations du détecteur de l'installation de la figure 1 ;
- les figures 3, 4, 5 et 6 sont des courbes illustrant la forme des signaux pouvant être obtenus en différents points de l'unité de traitement d'informations de la figure 2, en présence et en l'absence d'un milieu diffusant ;
- la figure 7 est une vue schématique d'un équipement de mesure par réflectance du taux d'oxygénation de l'hémoglobine mettant en oeuvre un procédé selon l'invention ;
- la figure 8 est une vue schématique d'un équipement d'imagerie en transmittance mettant en oeuvre un procédé selon l'invention ;
- la figure 9 est une vue analogue à celle de la figure 2 illustrant une variante de l'unité de traitement d'informations du détecteur de l'installation de la figure 1 ; et
- la figure 10 est une vue schématique d'une variante d'une installation selon l'invention et associée à un organigramme de nature analogue à celui de la figure 2 illustrant la structure de l'unité de traitement d'informations détectées par cette variante d'installation.

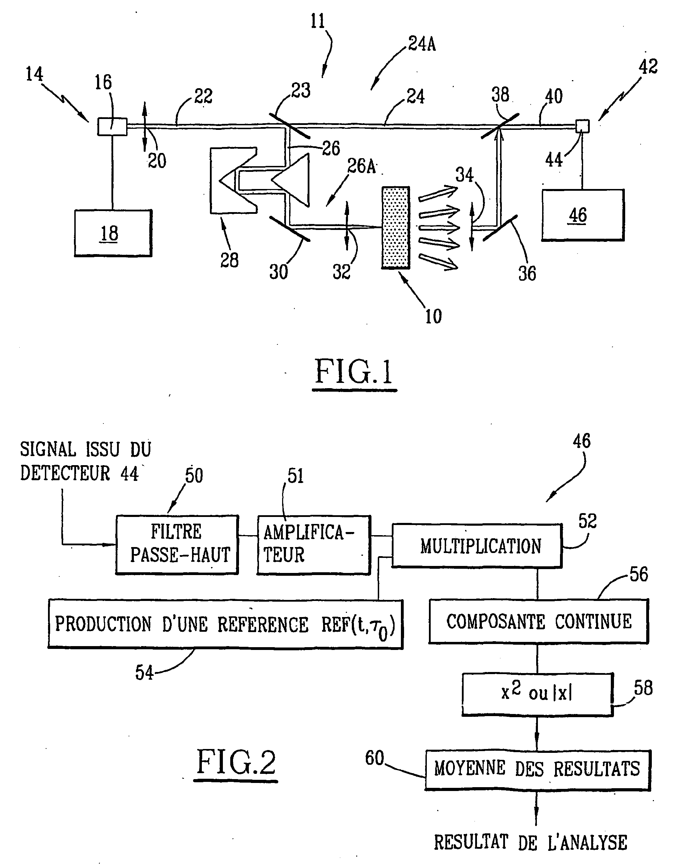

L'installation représentée sur la figure 1 illustre le principe du procédé d'analyse selon l'invention.

Cette installation permet l'analyse en transmittance d'un échantillon diffusant 10 par mesure résolue en temps de la lumière diffuse. Elle met en oeuvre une technique interférométrique pour effectuer la mesure et comporte à cet effet un interféromètre désigné par la référence générale 11.

A l'aide de l'installation, il est possible de reconstruire l'évolution temporelle de l'énergie diffuse avec des résolutions temporelles de l'ordre de 50 ps à 20 ps.

Plus précisément, l'installation met en oeuvre une source lumineuse 14 cohérente temporellement et modulée en longueur d'onde de façon à simuler, par un traitement approprié du signal d'interférence détecté, une source incohérente.

La source 14 comporte une cavité laser 16 reliée à une unité de commande 18 propre à assurer la modulation en longueur d'onde du faisceau produit par la cavité laser 16. La cavité laser 16 est par exemple une diode laser ou une diode laser montée en cavité étendue. Elle est adaptée pour produire un faisceau de lumière monochromatique cohérent temporellement.

La longueur d'onde centrale d'émission est choisie en fonction des applications. Suivant un mode particulier de mise en oeuvre du procédé, plusieurs longueurs d'onde centrales différentes sont successivement mises en oeuvre, permettant ainsi d'effectuer des mesures spectroscopiques. Elles sont par exemple égales à 780 nm et 850 nm pour l'analyse du taux d'oxygénation des tissus.

L'unité de commande 18, dont la réalisation pratique est à la portée de l'homme du métier, est adaptée pour une modulation périodique de la longueur d'onde du faisceau produit par la diode 16. Cette modulation est avantageusement une modulation sinusoïdale. L'amplitude $\delta\lambda$ de cette modulation est de l'ordre de quelques centièmes de nanomètre.

La résolution temporelle $\Delta t$ obtenue pour une amplitude $\delta\lambda$ de modulation de quelques centièmes de nanomètre est de l'ordre de $\Delta t = 2\lambda^2/(\pi c\delta\lambda)$, c'est-à-dire quelques dizaines de picosecondes.

Avantageusement, la modulation de la lumière produite par la diode 16 est effectuée de façon continue, en évitant les sauts de mode.

La fréquence f de modulation est choisie suffisamment rapide pour que le milieu diffusant puisse être considéré comme immobile pendant la période de modulation $f^{-1}$. La fréquence de modulation est avantageusement comprise entre 0,1 et 10 kHz et est par exemple choisie égale à 1 kHz.

En sortie de la diode 16 peut être prévue une lentille 20 assurant le réglage de la largeur du faisceau incident produit par la diode. Le faisceau incident, noté 22, est envoyé vers l'interféromètre 11 qui comporte, en entrée, un miroir semi-réfléchissant 23 assurant la séparation du faisceau incident 22 en deux faisceaux 24 et 26. Ces deux faisceaux 24, 26 se propagent respectivement suivant un bras 24A de référence de l'interféromètre et suivant un autre bras 26A contenant l'échantillon.

Dans le bras 26A, le faisceau 26 est d'abord envoyé dans un système optique connu en soi permettant d'ajuster la différence de marche entre les chemins optiques des deux bras de l'interféromètre. En sortie du système d'ajustement 28, le faisceau 26 est envoyé par un miroir 30 sur l'échantillon 10 formant le milieu diffusant. Une lentille 32 peut avantageusement assurer la focalisation du faisceau en un point donné de l'échantillon.

Des moyens 34 de collection du faisceau diffusé tels qu'une lentille convergente recueillent le faisceau en sortie de l'échantillon 10. La lumière diffusée est ensuite envoyée par un miroir 36 vers un miroir semi-réfléchissant 38 assurant la superposition du faisceau diffusé et du faisceau 24 se propageant suivant le bras de référence de l'interféromètre.

Avantageusement, l'interféromètre 11 comporte des moyens permettant de modifier légèrement en continu la position du faisceau 26 arrivant sur l'échantillon 10. La modification de la position du faisceau est effectuée au cours de l'analyse, afin d'augmenter la précision de celle-ci, comme cela sera expliqué ultérieurement.

Ces moyens comportent par exemple des organes de mise en mouvement du miroir 30 et/ou de la lentille convergente 32, afin de modifier la position du faisceau sur l'échantillon et/ou l'angle d'incidence du faisceau par rapport à l'échantillon.

De même, l'interféromètre comporte avantageusement, en aval de l'échantillon 10 dans le bras 26A, des moyens permettant de modifier sur l'échantillon la zone de recueil de la lumière diffuse. Ces moyens comportent par exemple des organes de mise en mouvement des moyens 34 de collecte ou du miroir 36.

Le faisceau noté 40 obtenu par superposition, en sortie de l'interféromètre 11, est envoyé à des moyens 42 de détection et d'analyse.

Ces moyens 42 comportent un détecteur 44 relié à une unité de traitement d'informations 46 dont les moyens principaux sont décrits en regard de la figure 2.

Le détecteur 44 est par exemple une photodiode.

Avantageusement, les caractéristiques des systèmes optiques placés dans le bras 26A de l'interféromètre, en aval de l'échantillon 10, sont choisies de façon à ce que la surface de cohérence du faisceau diffusé au niveau du détecteur 44 soit du même ordre de grandeur que la surface active de ce détecteur.

Dans le mode de réalisation décrit, l'unité de traitement d'informations 46 est formée d'un ensemble de circuits électroniques analogiques propres à assurer les fonctions décrites en regard de la figure 2.

En entrée, l'unité 46 comporte un filtre passe-haut 50 pour filtrer le signal recueilli par le détecteur 44. La fréquence de coupure du filtre 50 est de l'ordre de une à dix fois la fréquence f de modulation de la lumière émise par la diode 16. Dans le cas présent, la fréquence de coupure est de l'ordre du kilo hertz.

Le filtre passe-haut 50 a pour objectif principal de supprimer la composante continue du signal. Il assure également l'élimination des parasites de basse fréquence ainsi que les éventuels effets parasites liés à la modulation en longueur d'onde de la diode 16. Le signal d'interférence obtenu en sortie du filtre 50 a une fréquence beaucoup plus élevée que la fréquence de modulation.

Le signal obtenu en sortie du filtre est avantageusement amplifié par un amplificateur 51 facultatif.

Bien que le filtre 50 soit avantageusement mis en oeuvre, celui-ci peut être omis.

Un étage de multiplication 52 est prévu en sortie de l'amplificateur 51. Il assure la multiplication du signal filtré par un signal de référence $\text{Ref}(t,\tau_0)$. Le signal $\text{Ref}(t,\tau_0)$ est issu d'un dispositif 54 de production de ce signal. Ce dispositif est par exemple un générateur programmable de signaux ou un circuit électronique analogique constitué d'un ensemble d'oscillateurs dont les paramètres caractéristiques peuvent être ajustés.

La sortie de l'étage de multiplication 52 est reliée à un étage 56 d'extraction de la composante continue du signal, c'est-à-dire de calcul de sa valeur moyenne.

Le signal $\text{Ref}(t,\tau_0)$ est tel que la composante continue du produit du signal de référence $\text{Ref}(t,\tau_0)$ et du signal mesuré par le détecteur 44, en l'absence de milieu diffusant dans le second bras 26A de l'interféromètre est essentiellement nul, sauf si le retard $\delta t$ entre les deux bras de l'interféromètre est approximativement égal à $\tau_0$, à $\Delta t$ près, $\Delta t$ étant la résolution temporelle. Le retard $\delta t$ correspond à la différence de longueur de chemin optique entre les deux bras de l'interféromètre 11 en l'absence d'échantillon diffusant, divisée par la célérité de la lumière.

La combinaison du signal d'interférence et du signal de référence $\text{Ref}(t,\tau_0)$ agit comme une porte temporelle centrée sur le retard $\tau_0$, cette porte sélectionnant la composante du signal d'interférence correspondant un retard $\tau_0$ par rapport au temps de parcours du bras de référence 24A de l'interféromètre.

Le signal $\text{Ref}(t,\tau_0)$ s'exprime par exemple sous la forme :

$$\text{ref}(t,\tau_0) = A\,\sin^n(2\pi f\,t)\cos(\delta\lambda_0\tau_0\cos(2\pi f\,t)+\varphi)$$

pour une modulation de la lumière émise par la diode 16 s'exprimant sous la forme $\delta\lambda(t) = \delta\lambda_0\cos(2\pi f\,t)$.

Plusieurs valeurs de A, n et $\varphi$ sont possibles. Par exemple, les valeurs suivantes conviennent : A = 1, n = 4 et $\varphi$ = 0.

Suivant une variante de réalisation, le signal Ref(t,$\tau_0$) est produit en utilisant un interféromètre optique dépourvu d'échantillon et ayant un retard $\tau_0$ entre les deux bras, en filtrant la composante continue du signal détecté en sortie de l'interféromètre et en multipliant le résultat par une autre fonction générée par un dispositif électronique analogique, cette fonction s'annulant aux extrema de la modulation $\delta\lambda(t)$. Cette autre fonction sera par exemple $\sin^n(2\pi ft)$.

L'extraction par l'étage 56 de la composante continue du produit calculé par l'étage 52 est obtenue par exemple par mise en oeuvre d'un filtre passe-bas. Dans ce cas, le signal extrait est un signal continu dont l'évolution est une évolution lente liée aux mouvements de l'échantillon 10 ou aux mouvements des systèmes optiques 30, 32 et/ou 34, 36.

En variante, l'extraction de la composante continue est assurée par un dispositif intégrateur qui intègre le signal obtenu en sortie de l'étage 52 pendant exactement un nombre entier de demi-périodes $f^{-1}/_2$. Dans ce cas, le signal issu de l'étage 56 est échantillonné après chaque intégration de ce type.

L'unité de traitement d'informations comporte en outre un étage 58 d'application d'une fonction non linéaire sur la composante continue du signal obtenu à l'issue de l'étage 58. Cette fonction est, par exemple, une élévation au carré, ou à toute autre puissance paire, ou encore l'application de la fonction valeur absolue.

L'application d'une telle fonction conduit à considérer la seule amplitude des signaux, indépendamment de leur signe et de permettre que leur moyenne ne soit pas nulle.

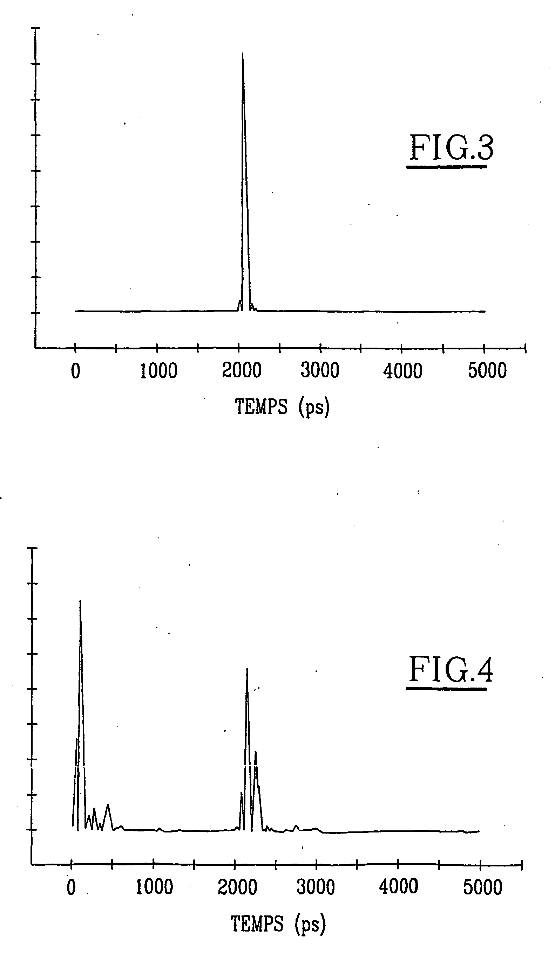

Le résultat de l'application de la porte temporelle mise en oeuvre lors de la multiplication effectuée par les étages 52 à 58 est illustré sur les figures 3 et 4. Le signal représenté sur ces figures est la composante continue obtenue en sortie de l'étage 58, en fonction de la valeur du retard $\tau_0$ prédéterminé.

Le signal représenté sur la figure 3 est obtenu à partir de l'installation de la figure 1, dans laquelle aucun échantillon n'est disposé dans le bras 26A.

Dans l'exemple considéré sur cette figure, le sommet du pic correspond à un retard sensiblement égal à 2080 ps, cette valeur correspondant au retard $\delta t$ entre les deux bras de l'interféromètre.

Au contraire, le signal représenté sur la figure 4 est obtenu, en sortie de l'étage 56, en présence d'un milieu diffusant disposé dans la branche 26A de l'interféromètre.

Sur cette courbe, on constate la présence d'un signal non nul au voisinage de la valeur $\delta t$ du retard entre les deux bras de l'interféromètre.

On constate également sur cette figure un premier signal proche de l'origine des temps, ce signal correspondant uniquement à la lumière diffuse provenant du bras 26A, indépendamment des interférences avec la référence provenant du bras 24A. Ce signal est non utilisé pour la mise en oeuvre du procédé.

L'étage suivant noté 60 de l'unité 46 est adapté pour établir la moyenne des signaux obtenus en sortie de l'étage 58 pendant une durée déterminée. La moyenne calculée par l'étage 60 est déterminée sur une durée de l'ordre de la seconde. A cet effet, l'étage 60 comporte par exemple un filtre passe-bas.

En variante, le calcul de la moyenne est remplacé par une simple sommation des signaux échantillonnés provenant de l'étage 58 ou tout autre forme de combinaison linéaire effectuée sur ces signaux.

Le signal obtenu à l'issue de l'étage 60 pour une valeur donnée du retard prédéterminé $\tau_0$ est proportionnel à la partie de l'énergie diffuse qui a un retard $\tau_0$ par rapport au temps de parcours du bras de référence 24A de l'interféromètre, à $\Delta t$ près.

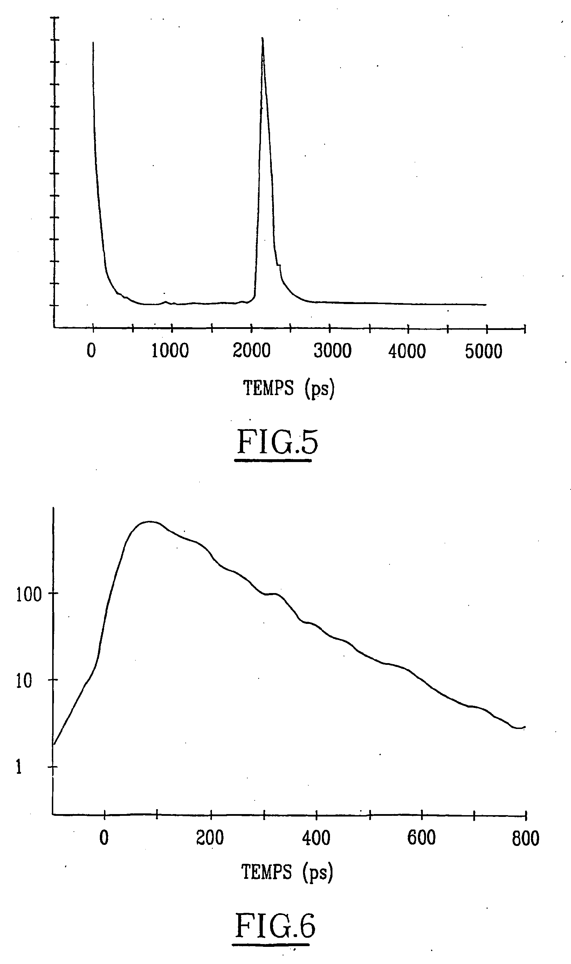

Sur la figure 5 est représenté un exemple du signal obtenu en sortie de l'étage 60, en fonction du retard $\tau_0$ prédéterminé. Cette figure montre clairement un signal pour des valeurs du retard $\tau_0$ prédéterminé supérieures au retard $\delta t$ entre les deux bras de l'interféromètre.

Du fait de la moyenne effectuée à l'issue de l'application d'une fonction non linéaire sur les signaux obtenus en sortie de la porte temporelle, le profil de la courbe de la figure 5 présente un profil net représentatif de la structure du milieu diffusant.

Le retard $\tau_0$ permet de déterminer le temps nécessaire au faisceau pour traverser l'échantillon, par exemple, en retranchant simplement du retard $\tau_0$ le temps de retard $\delta t$ entre les deux bras de l'interféromètre, et en ajoutant une correction liée à l'épaisseur de l'échantillon.

Le signal de la figure 5 est représenté à plus grande échelle sur la figure 6, où l'origine des temps a été corrigée du retard $\delta t$ entre les deux bras de l'interféromètre.

L'évolution de la valeur moyenne calculée est liée à une transformation des paramètres pertinents dans le milieu étudié.

Suivant un mode particulier de réalisation, l'installation comporte plusieurs unités de traitement d'informations 46, chacune associée à une valeur de retard $\tau_0$, permettant ainsi d'obtenir des mesures de l'énergie diffuse pour des retards $\tau_0$ différents.

L'unité de traitement d'informations 46, décrite précédemment, est constituée d'un dispositif électronique analogique. Toutefois, en variante, cette unité de traitement d'informations comporte un filtre passe-haut 50 analogique en sortie duquel est prévu un convertisseur analogique/numérique. L'ensemble des traitements effectués par les étages 52 à 60 est ensuite réalisé de manière complètement numérique par mise en oeuvre d'un algorithme adapté dans un calculateur. Dans ce cas, le nombre différent de valeurs $\tau_0$ pour lesquelles le calcul est effectué peut être très grand.

Pendant la mesure, le signal obtenu en sortie de l'étage 58 fluctue sur la même échelle de temps que le signal obtenu en sortie de l'étage 56. Ces fluctuations sont dues aux mouvements dans le milieu diffusant lui-même lorsque ce milieu est un milieu biologique. Ces mouvements induisent des fluctuations sur des échelles de temps de l'ordre de la milliseconde.

Or, ces fluctuations sont souhaitables pour permettre que la valeur moyenne obtenue en sortie de l'étage 60 soit aussi précise que possible. En effet, ces fluctuations permettent la mesure de valeurs différentes pour en faire la moyenne. Cette moyenne sera d'autant plus proche de sa valeur théorique, qui est l'intensité diffuse résolue en temps, que les fluctuations permettent d'obtenir un grand nombre de valeurs différentes.

Ainsi, avantageusement, si les fluctuations du milieu sont insuffisantes ou trop lentes, des fluctuations supplémentaires sont induites par mise en mouvement du faisceau 26 grâce au déplacement du miroir 30, de la lentille 32, des moyens de collection 34 et/ou du miroir 36.

La mise en mouvement est commandée de telle sorte que l'échelle de temps des fluctuations temporelles est plus grande que la période de modulation $f^{-1}$. Ainsi, la fréquence de déplacement du faisceau est inférieure à celle de la modulation en, longueur d'onde de la lumière incidente.

Une installation d'observation mettant en oeuvre un interféromètre, et des moyens d'émission d'un faisceau lumineux cohérent temporellement et modulé en longueur d'onde, ainsi que des moyens de traitement tels que décrits précédemment, peut être avantageusement mise en oeuvre pour la mesure par réflectance du taux d'oxygénation de l'hémoglobine d'un être humain.

Pour la mesure de ce taux d'oxygénation, deux longueurs d'onde $\lambda 1$ et $\lambda 2$ sont nécessaires.

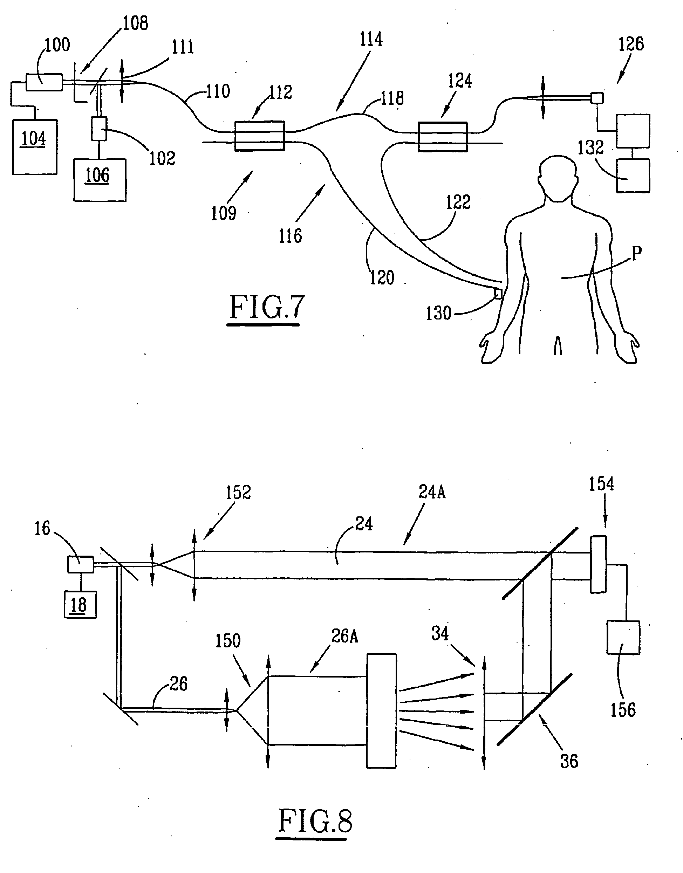

A cet effet, l'équipement de mesure du taux d'oxygénation illustré sur la figure 7 comporte une première diode laser 100 et une seconde diode laser 102 dont les longueurs d'onde centrales d'émission sont respectivement $\lambda 1$ et $\lambda 2$. Ces deux diodes sont commandées chacune par une unité de commande 104 et 106 permettant de moduler la longueur d'onde de la lumière émise, comme décrit dans le mode de réalisation précédent.

Un obturateur automatique 108 est disposé en sortie des diodes laser 100 et 102 afin d'illuminer sélectivement un interféromètre noté 109 avec l'un ou l'autre des faisceaux lumineux émis par ces diodes.

La sortie de l'obturateur automatique 108 est couplée à une fibre optique 110 par des moyens de couplage adaptés 111. La fibre 110 est une fibre optique monomode.

La fibre 110 achemine la lumière jusqu'à un coupleur 50/50 noté 112 permettant de séparer la lumière incidente et de diriger celle-ci suivant les deux bras notés 114 et 116 de l'interféromètre.

Le bras de référence 114 est formé par une fibre optique monomode 118.

Le second bras 116 comporte une fibre optique monomode 120 formant une fibre émettrice permettant d'amener le faisceau incident jusqu'à l'échantillon à étudier. Dans le cas présent, l'extrémité de la fibre optique est placée sur le bras d'un patient.

En aval de l'échantillon à étudier, le second bras 116 de l'interféromètre comporte une seconde fibre optique, notée 122, monomode constituant une fibre optique de collecte de la lumière diffuse. Son extrémité libre est placée à proximité de l'extrémité libre de la fibre émettrice 120 afin de recueillir la lumière diffuse rétrodiffusée.

Les fibres optiques 118 et 122 sont reliées l'une à l'autre par un coupleur 50/50, noté 124, permettant la superposition des faisceaux lumineux transmis dans les deux bras de l'interféromètre.

La sortie du coupleur 124 est reliée à des moyens de détection et d'analyse 126 analogues aux moyens 42 du mode de réalisation de la figure 1.

Avantageusement, un vibreur 130 facultatif est appliqué sur la fibre optique émettrice 120, et/ou la fibre réceptrice 122, afin de provoquer un léger déplacement de celle-ci au cours de l'analyse pour provoquer des fluctuations du signal et ainsi améliorer, comme expliqué précédemment, la précision de la mesure effectuée.

De préférence, pour chaque longueur d'onde $\lambda 1$, $\lambda 2$, la lumière diffusée est mesurée pour différents retards $\tau_0$, comme cela est décrit en regard du mode de réalisation de la figure 1.

L'équipement comporte, reliée au moyens de détection et d'analyse 126, une unité 132 de calcul du taux d'oxygénation de l'hémoglobine.

Le taux d'oxygénation de l'hémoglobine est déduit des coefficients d'absorption du milieu calculés aux deux longueurs d'onde $\lambda 1$, $\lambda 2$ propres aux diodes 100 et 102. Le calcul du coefficient d'absorption est fait, en fonction d'une relation connue en soi, à partir de la décroissance exponentielle de la diffusion de la lumière en fonction du temps, cette décroissance étant définie à partir des différentes mesures effectuées à des retards $\tau_0$ différents.

Alors que les installations illustrées sur les figures 1 et 7 permettent seulement d'effectuer une mesure résolue en temps en un point considéré de l'échantillon, l'installation illustrée sur la figure 8 est un équipement d'imagerie permettant de réaliser une image en deux dimensions de l'échantillon étudié.

Cet équipement reprend une structure sensiblement analogue à celle de l'installation de la figure 1. Ainsi, les éléments identiques ou analogues à ceux de ce mode de réalisation sont désignés par les mêmes numéros de référence.

Dans cet équipement d'imagerie, le bras 26A de l'interféromètre dans lequel est placé l'échantillon comporte, en amont de l'échantillon, un télescope 150 permettant l'élargissement du faisceau 26 se propageant suivant de bras.

Ainsi, l'essentiel de la surface de l'échantillon se trouve illuminé par le faisceau incident.

De même, le bras de référence 24A de l'interféromètre présente un télescope 152 agrandissant également le diamètre du faisceau 24 se propageant suivant ce bras.

Le capteur ponctuel 44 utilisé dans le mode de réalisation est ici remplacé par un réseau de capteurs ou pixels indépendants 154. Ce réseau comporte un ensemble de capteurs répartis en ligne et en colonne. Chaque capteur de détection est associé à un circuit intégré qui forme le filtre passe-haut 50 et amplifie le signal. Le signal issu de chaque capteur ainsi filtré et amplifié est ensuite multiplié par le signal de référence $Ref(t,\tau_0)$ puis intégré sur un nombre entier de demi-périodes pour en extraire la composante continue. Après quoi, les étapes de calcul d'une fonction non linéaire et de la moyenne des résultats de cette fonction sont effectuées avantageusement numériquement pour chaque capteur.

L'ensemble des résultats obtenus pour chaque capteur est envoyé à une unité 156 adaptée pour produire une image de l'échantillon étudié en fonction des résultats reçus.

La valeur du retard $\tau_0$ est avantageusement ajustée de façon à obtenir une image contrastée en sélectionnant la lumière diffuse aux temps courts.

Avantageusement, le télescope 150 et le système collecteur 34, 36 sont associés à des moyens permettant de modifier leur inclinaison par rapport à un axe de référence afin d'obtenir des images en transmittance selon différentes directions et ainsi permettre une reconstitution tridimensionnelle du milieu à partir des images obtenues de l'échantillon pour différentes positions du télescope 152 et du système collecteur 34, 36.

L'équipement de la figure 8 est destiné en particulier à l'observation par transmittance d'un organe d'un être humain ou animal. Il est utile notamment pour la recherche de tumeurs cancéreuses du sein.

Les installations décrites ici et le procédé qu'elles mettent en oeuvre permettent d'analyser un échantillon diffusant par mesure résolue en temps de la lumière diffuse dans cet échantillon pour un coût très réduit.

En effet, la source lumineuse utilisée peut être formée d'une simple diode laser. De même, les moyens de détection et d'analyse peuvent être réalisés à moindre coût.

De plus, avec la lumière modulée en longueur d'onde utilisée, les champs électromagnétiques sont beaucoup moins intenses qu'en régime impulsionnel, comme c'est le cas dans l'état de la technique.

Enfin, une très grande sensibilité peut être obtenue grâce à la nature interférométrique de l'analyse.

Même avec une simple photodiode comme détecteur, une très grande sensibilité et une très grande dynamique peuvent être obtenues.

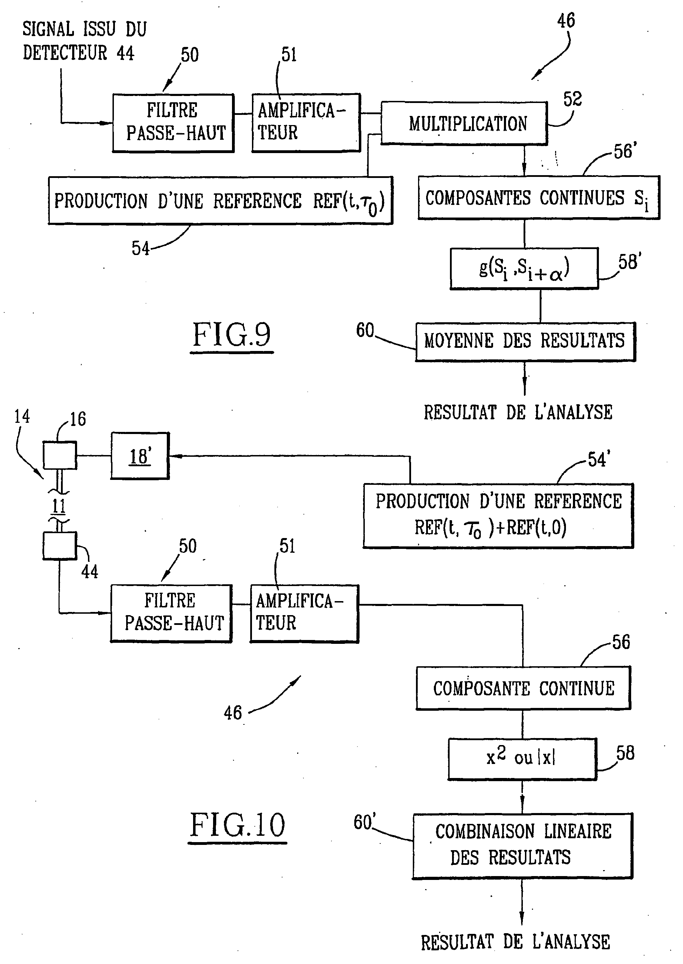

Sur la figure 9 est illustrée une variante du procédé d'analyse selon l'invention destinée à obtenir une mesure résolue en temps d'une quantité relative à la lumière diffuse dans l'échantillon à analyser. Cette variante se distingue du procédé décrit en regard des figures 1 à 6 uniquement par la structure de l'unité de traitement d'informations 46 au niveau de ses étages 56 et 58.

La sortie de l'étage de multiplication 52 est reliée à un étage 56' d'extraction des composantes continues respectives des signaux obtenus en sortie de l'étage 52 pendant une durée déterminée, sensiblement égale à celle retenue pour la détermination de la moyenne calculée par l'étage 60 dans le procédé décrit en regard de la figure 2. Les composantes continues ainsi obtenues à l'étage 56' sont notées $S_i$, où i désigne le $i^{ème}$ signal en sortie de l'étage 52 pendant la durée précitée. Les moyens mis en oeuvre pour obtenir chaque composante continue $S_i$ sont analogues à ceux explicités précédemment.

La sortie de cet étage 56' est reliée à un étage 58' d'application d'une fonction g non linéaire à au moins deux variables prises parmi les composantes continues $S_i$ issues de l'étage 56'. Il est à noter que le terme « non linéaire » est considéré ici dans un sens large, c'est-à-dire, dans le cas de fonctions à plusieurs variables, seules sont exclues les fonctions linéaires par rapport à leurs variables prises ensemble sous forme vectorielle, comme la fonction « somme de deux vecteurs » par exemple.

Cette fonction non linéaire est, par exemple, une fonction produit appliquée à deux valeurs $S_i$ distinctes. Ainsi, on peut considérer la fonction $g(S_i, S_{i+\alpha}) = S_i \times S_{i+\alpha}$ où $\alpha$ est un entier positif non nul. Une telle fonction est adaptée pour étudier l'évolution de la valeur $S_{i+\alpha}$ par rapport à la valeur $S_i$. En effet, comme il a été expliqué précédemment, un milieu biologique est le siège de nombreux mouvements et, malgré les précautions d'usage consistant notamment à choisir une fréquence de modulation de la source laser 14 suffisamment élevée pour considérer le milieu diffusant 10 comme immobile pendant la période de modulation, le signal $S_{i+1}$ enregistré à la suite d'un signal $S_i$ est légèrement différent de ce signal $S_i$. Le signal $S_{i+2}$ l'est davantage. Plus généralement, on note une décorrélation des signaux $S_{i+\alpha}$ et $S_i$ pour $\alpha \geq 1$. L'évolution de cette décorrélation en fonction de $\alpha$ et de $\tau_0$ est à même de fournir des données utiles sur la nature et l'intensité des mouvements dans le milieu analysé 10.

A cet effet, la sortie de l'étage 58' est reliée à l'étage 60 de moyenne des signaux obtenus en sortie de l'étage 58', laquelle moyenne est fonction, d'une part, du retard $\tau_0$ comme précédemment, et d'autre part de la valeur de l'entier $\alpha$. Un moyen d'étudier la décorrélation des signaux $S_i$ entre eux consiste à suivre l'évolution de cette moyenne issue de l'étage 60, pour $\alpha$ croissant et $\tau_0$ fixé par exemple.

Dans le cas de la mesure du taux d'oxygénation de l'hémoglobine d'un être humain illustrée sur la figure 7, les données relatives à la décorrélation des signaux $S_i$ renseignent sur la circulation sanguine dans des tissus tels que les capillaires du muscle, en complément de la mesure du taux d'oxygénation comme expliqué en regard de la figure 7. L'acquisition résolue en temps de ces données permet par exemple d'isoler la contribution de la circulation sanguine dans de tels capillaires par rapport à celle dans des vaisseaux sanguins de tailles plus importantes.

Dans le cas de la recherche de tumeurs cancéreuses illustrée sur la figure 8, les données relatives à la décorrélation des signaux $S_i$ renseignent sur l'état de vascularisation d'une tumeur observée par l'équipement de la figure 8. Généralement, de telles tumeurs sont sur-vascularisées. Le contraste de l'image de l'échantillon étudié peut être ainsi amélioré en utilisant ces données de décorrélation.

Sur la figure 10 est illustrée une seconde variante du procédé d'analyse selon l'invention dans laquelle les moyens de combinaison du signal d'interférence avec la référence Ref (t, $\tau_0$) sont constitués par des moyens de modulation en amplitude de la source laser 16. Plus précisément, cette variante de la figure 10 ne se distingue du mode de réalisation décrit en regard des figures 1 et 2 que par ce qui suit.

D'abord, l'unité de commande 18 est remplacée par une unité de commande 18' adaptée à la fois pour moduler en longueur d'onde le faisceau produit par la cavité laser, sensiblement de façon analogue à l'unité 18 de la figure 1, et pour moduler en amplitude ce faisceau laser, de façon indépendante, à partir d'un signal extérieur de modulation en amplitude. La réalisation d'une telle unité de commande 18' est à la portée de l'homme du métier, par exemple dans le cas de l'utilisation d'une diode laser 16 montée en cavité étendue.

Selon l'invention, cette modulation supplémentaire en amplitude est capable d'introduire dans l'installation d'analyse le signal de référence Ref (t, $\tau_0$). A cet effet, un dispositif 54' de production d'un signal Ref (t, $\tau_0$) + Ref (T, 0) est prévu, de nature sensiblement analogue au dispositif 54 de la figure 2. Ce dispositif 54' est relié à l'unité de commande 18' pour fournir le signal extérieur de modulation en amplitude.

A la sortie des étages de filtrage 50 et d'amplification 51, le signal d'interférence enregistré se présente alors directement sous la forme d'une combinaison, notamment d'un produit, comportant d'une part un premier terme de nature analogue à celui issu des étages 50 et 51 de la figure 1, c'est-à-dire un premier terme porteur de l'information modulée en longueur d'onde, et d'autre part un second terme de nature analogue à celui issu du dispositif 54 de la figure 1, c'est-à-dire un second terme porteur du retard $\tau_0$. Le signal combiné ainsi obtenu est caractéristique du retard $\tau_0$.

Par ailleurs; l'étage 60 de moyenne des signaux issus de l'étage 58 de la figure 1 est remplacé par un étage 60' adapté pour à la fois réaliser une telle moyenne, et pour soustraire du résultat obtenu, la valeur moyenne correspondant au traitement par les étages 56 et 58 d'un signal caractéristique d'un retard $\tau_0 = 0$, c'est-à-dire d'un retard nul. Cet étage 60' ne réalise donc qu'une combinaison linéaire des résultats issus de l'étage 58.

De cette façon, les résultats à la sortie de l'étage 60' sont identiques à ceux issus de l'étage 60 de la figure 2.

Cette variante permet de simplifier l'unité de traitement 56 des signaux d'interférence. Par exemple, pour l'équipement d'imagerie de la figure 8, cette variante permet de simplifier le réseau de capteurs 154, chaque capteur étant associé à un circuit intégré qui filtre, amplifie et extrait la composante continue, avec une acquisition sur une demi-période, de chaque signal d'interférence. Il est alors possible d'utiliser une caméra CCD comme moyen 156 pour produire une image de l'échantillon étudié.

Une autre variante non représentée du procédé d'analyse selon l'invention consiste à combiner ensemble les deux variantes des figures 9 et 10 et notamment dans la variante de la figure 10 à remplacer les étapes 56, 58 par les étapes 56', 58'.

Par ailleurs, divers aménagements du montage interférométrique 11 sont envisageables, dans la mesure où ils relèvent des connaissances de l'homme du métier. Par exemple, le miroir de superposition 38 peut produire deux faisceaux 40, chacun de ces deux faisceaux étant détecté par un capteur respectif 44. La différence obtenue entre ces deux faisceaux détectés donne une information analogue à celle issue précédemment de l'unique capteur, mais avantageusement moins bruitée.

**Revendications**

1. Procédé d'analyse d'un échantillon diffusant par mesure résolue en temps de la lumière diffuse dans cet échantillon, comportant les étapes de :

   a) illuminer l'échantillon avec un faisceau incident de lumière cohérente temporellement et modulée en longueur d'onde ;
   b) produire une succession de signaux d'interférence, enregistrés sur un intervalle de temps, par superposition de la lumière diffuse obtenue en sortie de l'échantillon et de lumière prélevée sur le faisceau incident illuminant l'échantillon ;
   c) combiner chaque signal d'interférence avec un signal de référence Ref(t,$\tau_0$), générant une porte temporelle centrée sur un retard $\tau_0$ prédéterminé pour produire un signal caractéristique du retard $\tau_0$ prédéterminé ;
   d) extraire la composante continue de chaque signal caractéristique du retard $\tau_0$ prédéterminé ;

e) appliquer une fonction non linéaire sur chacune des composantes continues des signaux caractéristiques du retard $\tau_0$ prédéterminé ; et

f) effectuer une combinaison linéaire de chacune des images, après application de ladite fonction non linéaire, des composantes continues des signaux caractéristiques du retard $\tau_0$ prédéterminé.

**2.** Procédé selon la revendication 1, où ladite fonction non linéaire est une fonction choisie dans le groupe consistant en l'élévation au carré, et la fonction valeur absolue.

**3.** Procédé selon la revendication 1, où ladite fonction non linéaire est une fonction à au moins deux variables prises parmi les composantes continues des signaux caractéristiques du retard $\tau_0$ prédéterminé obtenus à partir de la succession des signaux d'interférence enregistrés sur l'intervalle de temps.

**4.** Procédé selon l'une quelconque des revendications précédentes, dan lequel pour la combinaison de chaque signal d'interférence avec ledit signal de référence Ref(t,$\tau_0$) le faisceau incident est modulé, de façon supplémentaire, en amplitude au moyen d'une modulation en amplitude introduisant le retard $\tau_0$ prédéterminé.

**5.** Procédé selon la revendication 4, dans lequel, pour une modulation en amplitude valant la somme dudit signal de référence Ref(t,$\tau_0$) et d'un signal de référence à retard nul Ref(t, 0), ladite combinaison linéaire comporte un calcul de soustraction de chacune des images, ladite fonction non linéaire, des composantes continues des signaux caractéristiques d'un retard nul

**6.** Procédé selon l'une quelconque des revendications précédentes, où ladite combinaison linéaire comporte un calcul de la moyenne de chacune des images après application de ladite fonction non linéaire des composantes continues des signaux caractéristiques du retard $\tau_0$ prédéterminé.

**7.** Procédé selon l'une quelconque des revendications précédentes, dans lequel pendant la production de la succession de signaux d'interférence, un déplacement relatif est imposé entre le faisceau incident et l'échantillon diffusant, la fréquence du déplacement imposé étant inférieure à la fréquence de la modulation en longueur d'onde de la lumière du faisceau incident.

**8.** Procédé selon l'une quelconque des revendications précédentes, où ledit signal de référence Ref(t,$\tau_0$) comporte une fonction sinusoïdale du produit du retard $\tau_0$ prédéterminé sur lequel est centrée la porte temporelle et de la fonction de modulation en longueur d'onde du faisceau incident.

**9.** Installation d'analyse d'un échantillon diffusant par mesure résolue en temps de la lumière diffuse dans cet échantillon, comportant:

a) des moyens (16, 18 ; 100, 104, 102, 106) pour illuminer l'échantillon avec un faisceau incident de lumière cohérente temporellement et modulée en longueur d'onde ;
b) un interféromètre (11 ; 109) pour produire une succession de signaux d'interférence, enregistrés sur un intervalle de temps, par superposition de la lumière diffuse obtenue en sortie de l'échantillon et de lumière prélevée sur le faisceau incident illuminant l'échantillon ;
c) des moyens (52, 54) pour combiner chaque signal d'interférence avec un signal de référence Ref(t,$\tau_0$) générant une porte temporelle centrée sur un retard $\tau_0$ prédéterminé pour produire un signal caractéristique du retard $\tau_0$ prédéterminé;
d) des moyens (56) pour extraire la composante continue de chaque signal caractéristique du retard $\tau_0$ prédéterminé ;
e) des moyens (58) pour appliquer une fonction non linéaire sur chacune des composantes continues des signaux caractéristiques du retard $\tau_0$ prédéterminé ; et
f) des moyens (60) pour effectuer une combinaison linéaire de chacune des images, après application de ladite fonction non linéaire, des composantes continues des signaux caractéristiques du retard $\tau_0$ prédéterminé.

**10.** Equipement de mesure de la teneur en un constituant déterminé dans une partie d'un organisme vivant comportant une installation d'analyse d'un échantillon diffusant selon la revendication 9, et des moyens (132) de calcul de ladite teneur en un constituant déterminé en fonction du résultat de l'analyse fourni par ladite installation.

**11.** Equipement selon la revendication 10, où ladite installation d'analyse est adaptée pour produire des signaux d'interférence à partir de la lumière rétrodiffusée par un organe d'un être humain ou d'un animal, et en ce que lesdits moyens de calcul sont adaptés pour calculer le taux d'oxygénation de cet organe.

12. Equipement d'imagerie comportant :

   - une installation d'analyse selon la revendication 9, laquelle installation est adaptée pour produire et traiter plusieurs successions de signaux d'interférence obtenus à partir de la lumière diffuse en plusieurs régions adjacentes de l'échantillon et ainsi fournir un résultat d'analyse pour chacune des régions adjacentes de l'échantillon ; et
   - des moyens (156) pour produire une image de l'échantillon à partir du résultat d'analyse fourni pour chacune des régions adjacentes de l'échantillon.

13. Equipement selon la revendication 12, où ladite installation d'analyse est adaptée pour produire des signaux d'interférence à partir de la lumière diffuse transmise au travers d'un organe d'un être humain ou d'un animal.

**Claims**

1. Method of analysing a diffusing sample by time resolution measurement of the diffused light in this sample, comprising the steps of:

   a) illuminating the sample with an incident beam of light which is coherent temporally and modulated in wavelength;
   b) producing a succession of interference signals, recorded over a time interval, by superimposing the diffused light obtained on exit from the sample and the light sampled from the incident beam illuminating the sample;
   c) combining each interference signal with a reference signal Ref(t, $\tau_0$), generating a temporal gate which is centred on a predetermined delay $\tau_0$ in order to produce a signal which is characteristic of the predetermined delay $\tau_0$;
   d) extracting the continuous component of each signal which is characteristic of the predetermined delay $\tau_0$;
   e) applying a non-linear function to each of the continuous components of the signals which are characteristic of the predetermined delay $\tau_0$; and
   f) effecting a linear combination of each of the images, after applying said non-linear function, from the continuous components of the signals which are characteristic of the predetermined delay $\tau_0$.

2. Method according to claim 1, where said non-linear function is a function chosen from the group comprising raising to a square and the absolute value function.

3. Method according to claim 1, where said non-linear function is a function with at least two variables taken from amongst the continuous components of the signals which are characteristic of the predetermined delay $\tau_0$ and obtained from the succession of interference signals recorded over the time interval.

4. Method according to any of the preceding claims, in which, for the combination of each interference signal with said reference signal Ref(t, $\tau_0$), the incident beam is modulated, in an additional manner, in amplitude, by means of an amplitude modulation introducing the predetermined delay $\tau_0$.

5. Method according to claim 4, in which, for a modulation in amplitude equal to the sum of said reference signal Ref(t, $\tau_0$) and of a reference signal with a zero delay Ref(t, 0), said linear combination comprises a calculation of subtracting each of these images, after application of said non-linear function, from the continuous components of the signals which are characteristic of a zero delay.

6. Method according to any of the preceding claims, where said linear combination comprises a calculation of the mean of each of the images, after application of said non-linear function, from the continuous components of the signals which are characteristic of the predetermined delay $\tau_0$.

7. Method according to any of the preceding claims, in which, during production of the succession of interference signals, a relative displacement is imposed between the incident beam and the diffusing sample, the frequency of the displacement imposed being less than the frequency of the wavelength modulation of the incident light beam.

8. Method according to any of the preceding claims, where said reference signal Ref(t, $\tau_0$) comprises a sinusoidal

function of the product of the predetermined delay $\tau_0$ on which the temporal gate is centred and of the function of the wavelength modulation of the incident beam.

9.  Installation for analysing a diffusing sample by time resolution measurement of the diffused light in this sample, comprising:

   a) means (16, 18; 100, 104, 102, 106) for illuminating the sample with an incident beam of light which is coherent temporally and modulated in wavelength;
   b) an interferometer (11; 109) for producing a succession of interference signals, recorded over a time interval, by superimposing the diffused light obtained on exit from the sample and the light sampled from the incident beam illuminating the sample;
   c) means (52, 54) for combining each interference signal with a reference signal Ref(t, $\tau_0$), generating a temporal gate which is centred on a predetermined delay $\tau_0$ in order to produce a signal which is characteristic of the predetermined delay $\tau_0$;
   d) means (56) for extracting the continuous component of each signal which is characteristic of the predetermined delay $\tau_0$;
   e) means (58) for applying a non-linear function to each of the continuous components of the signals which are characteristic of the predetermined delay $\tau_0$; and
   f) means (60) for effecting a linear combination of each of the images, after applying said non-linear function, from the continuous components of the signals which are characteristic of the predetermined delay $\tau_0$.

10. Equipment for measuring the content of a specific constituent in a part of a living organism, comprising an installation for analysing a diffusing sample according to claim 9, and means (132) for calculating said content of a specific constituent as a function of the result of the analysis provided by said installation.

11. Equipment according to claim 10, where said analysis installation is adapted to produce interference signals from the light which is backscattered by an organ of a human being or of an animal, and in that said calculation means are adapted to calculate the oxygenation rate of this organ.

12. Imaging equipment comprising:

   - an analysis installation according to claim 9, which installation is adapted to produce and process a plurality of successions of interference signals which are obtained from the diffused light in a plurality of adjacent regions of the sample and thus to provide an analysis result for each of the adjacent regions of the sample; and
   - means (156) for producing an image of the sample from the analysis result provided for each of the adjacent regions of the sample.

13. Equipment according to claim 12, where said analysis installation is adapted in order to produce interference signals from the diffused light which is transmitted through an organ of a human being or of an animal.

**Patentansprüche**

1.  Verfahren zur Analyse einer lichtstreuenden Probe durch zeitaufgelöste Messung des Lichts, das in dieser Probe gestreut wird, das die folgenden Schritte umfasst:

   a) Beleuchten der Probe mit einem temporal kohärent und wellenlängenmoduliert einfallenden Lichtstrahl;
   b) Produzieren einer Reihe von Interferenzsignalen, die über ein Zeitintervall aufgezeichnet werden, durch die Überlagerung des Streulichts, das am Ausgang der Probe erhalten wird, und dem Licht, das am einfallenden Strahl, der die Probe beleuchtet, aufgenommen wird;
   c) Kombinieren jedes Interferenzsignals mit einem Referenzsignal, Ref(t,$\tau_0$), was ein Zeitgatter, gemittelt auf einer vorbestimmten Verzögerung $\tau_0$ erzeugt, um ein Signal zu produzieren, das für die vorbestimmte Verzögerung $\tau_0$ kennzeichnend ist;
   d) Extrahieren des Gleichstromanteils jedes Signals, der für die vorbestimmte Verzögerung $\tau_0$ kennzeichnend ist;
   e) Anwenden einer nichtlinearen Funktion auf jeden der Gleichstromanteile der Signale, die für die vorbestimmte Verzögerung $\tau_0$ kennzeichnend sind; und
   f) Ausführen einer linearen Kombination jedes der Bilder, nach dem Anwenden der nichtlinearen Funktion, der Gleichstromanteile der Signale, die für die vorbestimmte Verzögerung $\tau_0$ kennzeichnend sind.

EP 1 285 256 B1

2. Verfahren nach Anspruch 1, wobei die nichtlineare Funktion eine Funktion ist, ausgewählt aus der Gruppe bestehend aus der Quadratfunktion und der Absolutwertfunktion.

3. Verfahren nach Anspruch 1, wobei die nichtlineare Funktion eine Funktion mit mindestens zwei Variablen ist, die aus den Gleichstromanteilen der Signale gewählt sind, die für die vorbestimmte Verzögerung $\tau_0$ kennzeichnend sind, die aus der Reihe der Interferenzsignale erhalten werden, die über das Zeitintervall aufgezeichnet werden.

4. Verfahren nach einem der vorhergehenden Ansprüche, worin für die Kombination jedes Interferenzsignals mit dem Referenzsignal Ref$(t,\tau_0)$, der einfallende Strahl zusätzlich mittels einer Amplitudenmodulation amplitudenmoduliert ist, die die vorbestimmte Verzögerung $\tau_0$ einführt.

5. Verfahren nach Anspruch 4, worin für eine Amplitudenmodulation, die gleich der Summe des Referenzsignals Ref$(t,\tau_0)$ und eines Referenzsignals mit Verzögerung Null Ref$(t,\tau_0)$ ist, die lineare Kombination eine Berechnung umfasst, bei der jedes der Bilder nach Anwendung der nichtlinearen Funktion der Gleichstromanteile der Signale, die für eine Verzögerung Null kennzeichnend sind, subtrahiert wird.

6. Verfahren nach einem der vorhergehenden Ansprüche, wobei die lineare Kombination eine Berechnung des Mittelwerts jedes der Bilder nach Anwendung der nichtlinearen Funktion der Gleichstromanteile der Signale, die für die vorbestimmte Verzögerung $\tau_0$ kennzeichnend sind, umfasst.

7. Verfahren nach einem der vorhergehenden Ansprüche, worin, während der Produktion der Reihe von Interferenzsignalen, eine relative Verschiebung zwischen dem einfallenden Strahl und der lichtstreuenden Probe erzwungen wird, wobei die Frequenz der erzwungenen Verschiebung kleiner ist als die Frequenz der Wellenlängenmodulation des Lichts des einstrahlenden Strahls.

8. Verfahren nach einem der vorhergehenden Ansprüche, wobei das Referenzsignal Ref$(t,\tau_0)$ eine Sinusfunktion des Produkts der vorbestimmten Verzögerung $\tau_0$, auf dem das Zeitgatter gemittelt ist, und der Wellenlängenmodulationsfunktion des einfallenden Strahls umfasst.

9. Vorrichtung zur Analyse einer lichtstreuenden Probe durch zeitaufgelöste Messung des Lichts, das in dieser Probe gestreut wird, die umfasst:

a) Mittel (16, 18; 100, 104, 102, 106) zum Beleuchten der Probe mit einem temporal kohärent und wellenlängenmoduliert einfallenden Lichtstrahl;
b) ein Interferometer (11; 109) zum Produzieren einer Reihe von Interferenzsignalen, die über ein Zeitintervall aufgezeichnet werden, durch die Überlagerung des Streulichts, das am Ausgang der Probe erhalten wird, und dem Licht, das am einfallenden Strahl, der die Probe beleuchtet, aufgenommen wird;
c) Mittel (52, 54) zum Kombinieren jedes Interferenzsignals mit einem Referenzsignal, Ref$(t,\tau_0)$, was ein Zeitgatter, gemittelt auf einer vorbestimmten Verzögerung $\tau_0$, erzeugt, um ein Signal zu produzieren, das für die vorbestimmte Verzögerung $\tau_0$ kennzeichnend ist;
d) Mittel (56) zum Extrahieren des Gleichstromanteils jedes Signals, der für die vorbestimmte Verzögerung $\tau_0$ kennzeichnend ist;
e) Mittel (58) zum Anwenden einer nichtlinearen Funktion auf jeden der Gleichstromanteile der Signale, die für die vorbestimmte Verzögerung $\tau_0$ kennzeichnend sind; und
f) Mittel (60) zum Ausführen einer linearen Kombination jedes der Bilder nach dem Anwenden der nichtlinearen Funktion der Gleichstromanteile der Signale, die für die vorbestimmte Verzögerung $\tau_0$ kennzeichnend sind.

10. Einrichtung zum Messen des Gehalts an einem bestimmten Bestandteil in einem Teil eines lebenden Organismus, die eine Vorrichtung zur Analyse einer lichtstreuenden Probe nach Anspruch 9, und Mittel (132) zur Berechnung des Gehalts an einem bestimmten Bestandteil in Abhängigkeit vom Ergebnis der Analyse, die von der Vorrichtung bereitgestellt wird, umfasst.

11. Einrichtung nach Anspruch 10, wobei die Vorrichtung zur Analyse angepasst ist, Interferenzsignale zu produzieren, die von dem durch ein Organ eines Menschen oder eines Tiers rückgestreuten Lichts ausgehen, und **dadurch**, dass die Mittel zur Berechnung angepasst sind, um die Oxygenierungsrate dieses Organs zu berechnen.

12. Bildgebende Einrichtung, die umfasst:

- eine Vorrichtung zur Analyse nach Anspruch 9, wobei die Vorrichtung angepasst ist, mehrere Reihen von Interferenzsignalen zu produzieren und zu behandeln, die aus dem Streulicht in mehreren benachbarten Regionen der Probe erhalten wurden, und so ein Analyseergebnis für jede der benachbarten Regionen der Probe bereitzustellen; und
- Mittel (156), um ein Bild der Probe aus dem Analyseergebnis zu produzieren, das für jede der benachbarten Regionen der Probe bereitgestellt wird.

13. Einrichtung nach Anspruch 12, wobei die Vorrichtung zur Analyse angepasst ist, um Interferenzsignale aus dem Streulicht zu produzieren, das durch ein Organ eines Menschen oder eines Tieres transmittiert wird.

## FIG.1

FIG.3

TEMPS (ps)

FIG.4

TEMPS (ps)

TEMPS (ps)

## FIG.5

TEMPS (ps)

## FIG.6

FIG.7

FIG.8

SIGNAL ISSU DU
DETECTEUR 44

```
┌──────────────┐   ┌──────────────┐        ┌──────────────────┐
│    FILTRE    │   │  AMPLIFICA-  │        │   MULTIPLICATION │
│  PASSE-HAUT  │───│    TEUR      │────────│                  │
└──────────────┘   └──────────────┘        └──────────────────┘
      50                 51                        52
```

46

┌──────────────────────────────────────────┐   ┌──────────────────────────────┐
│ PRODUCTION D'UNE REFERENCE REF(t,$\tau_0$) │   │ COMPOSANTES CONTINUES $S_i$ │
└──────────────────────────────────────────┘   └──────────────────────────────┘
                    54                                        56'

┌────────────────────┐
│ $g(S_i, S_{i+\alpha})$ │   58'
└────────────────────┘

┌──────────────────────────┐
│  MOYENNE DES RESULTATS   │   60
└──────────────────────────┘

RESULTAT DE L'ANALYSE

## FIG.9

14  16

```
┌──┐     ┌──────┐
│  │─────│  18' │◄─────────────┐
└──┘     └──────┘              │
 ┃                   ┌──────────────────────────────┐
11                   │  PRODUCTION D'UNE REFERENCE   │   54'
 ┃                   │  REF(t, $\tau_0$)+REF(t,0)    │
┌──┐                 └──────────────────────────────┘
│  │
└──┘
  44
```

```
┌──────────────┐   ┌──────────────┐
│    FILTRE    │   │  AMPLIFICA-  │
│  PASSE-HAUT  │───│    TEUR      │────────┐
└──────────────┘   └──────────────┘        │
      50                 51                 │
```

46

┌──────────────────────────┐
│   COMPOSANTE CONTINUE     │   56
└──────────────────────────┘

┌──────────────────────────┐
│      $x^2$ ou $|x|$       │   58
└──────────────────────────┘

┌──────────────────────────┐
│  COMBINAISON LINEAIRE     │
│     DES RESULTATS         │   60'
└──────────────────────────┘

RESULTAT DE L'ANALYSE

## FIG.10

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

• US 5692511 A

**Littérature non-brevet citée dans la description**

• **D, HUANG et al.** *science,* 1991, vol. 254, 1178-1181